# EUROPEAN PATENT SPECIFICATION

(11) **EP 1 547 549 B1**
(45) Date of publication and mention of the grant of the patent: **26.08.2009**
(21) Application number: 04257892.2
(22) Date of filing: 17.12.2004
(51) Int. Cl.: A61F 5/00, A61B 17/12

(54) **Mechanically adjustable gastric band**
Mechanisch einstellbares Magenband
Bande gastrique réglable mécaniquement

(30) Priority: 17.12.2003 US 530497 P
(43) Date of publication of application: 29.06.2005
(73) Proprietor: ETHICON ENDO-SURGERY, INC., Cincinnati, Ohio 45242 (US)
(72) Inventor: Uth, Josh, Mason, Ohio45040 (US); Dlugos, Dan, Morrow, Ohio 45152 (US); Huitema, Tom, Cincinnati, Ohio 45240 (US); Conlon, Sean, Loveland, Ohio 45140 (US); Hassler, Bill, Cincinnati, Ohio 45249 (US); Ortiz, Mark, Milford, Ohio 45150 (US); Freeman, Lynn, West Chester, Ohio 45069 (US); McKenna, Rob, Cincinnati, Ohio 45241 (US); Byrum, Randy, Kings Mills, Ohio 45034 (US); Schulze, Dale, Lebanon, Ohio 45036 (US)
(74) Representative: Tunstall, Christopher Stephen

(56) References cited:
- WO-A-01/10359
- US-A- 4 592 339
- US-A- 5 938 669
- US-A- 6 067 991
- US-A1- 2001 011 543
- US-A1- 2003 066 536
- US-B1- 6 460 543
- US-B1- 6 547 801

## Description

### Background

The present invention relates to a food intake restriction device for the treatment of morbid obesity. More specifically, the invention relates to a food intake restriction device for surgical application in the abdomen of a patient for forming a stoma opening in the stomach or esophagus of the patient.

Surgeons have implanted gastric banding devices into morbidly obese patients for many years with significant success. In a typical gastric banding procedure, the surgeon carefully positions the gastric band around the upper stomach using proven surgical techniques to create a small, upper pouch of the stomach. The gastric band comprises a non-extendable portion that surrounds the stomach and an inflatable portion, or balloon, which may be filled with a fluid such as saline to adjust the amount of constriction on the stomach. Gastric bands of this type usually are permanent implants, but require periodic adjustment to accommodate patient needs related to the rate of weight loss, ability to swallow, and other general health considerations. The surgeon may adjust the band by filling or removing fluid in the balloon, using a hypodermic needle penetrated transcutaneously into an injection port implanted beneath the skin and fat layers of the abdomen or thorax.

A great disadvantage of repeatedly injecting fluid via the injection port is the increased risk of infection, perhaps requiring surgical intervention. Also, the patient may swallow pieces of food that are too large to pass the restricted stoma opening, sometimes requiring a doctor to enlarge the stoma opening by removing fluid from the band via the injection port. What is needed is an adjustable food intake restriction device, which permits regular, post-operative adjustments that are comfortable for the patient, and which do not increase the risk of infection.

### Summary of the Invention

The present invention is a food intake restriction device as recited in the claims.

### Brief Description of the Drawings

FIG. 1 is a top view of a first gastric band embodiment 12, which is actuated by rotation of a torsion cable 40;
FIG. 2 is a cut-away, top view of first gastric band embodiment 12, revealing an extendable shield 22;
FIG. 3 is an enlarged view of a portion of extendable shield 22 containing a first strand 24 and a second strand 26 twisted together;
FIG. 4 is a top view of a second gastric band embodiment 60 not according to the invention, which is electrically actuated by a band actuator 72 containing a motor 78 and a release mechanism 94;
FIG. 5 is a side view of second gastric band embodiment 60;
FIG. 6 is a top view of an implantable control port 110 for use with second gastric band embodiment 60;
FIG. 7 is a cross-sectional view of implantable control port 110, revealing a reservoir 116 and a control unit 118;
FIG. 8 is a representation of a control system 400 that may be adapted for use with the present invention, including an implanted portion 404 in a patient 2 and an external portion 402;
FIG. 9 is a third gastric band embodiment 126 not according to the invention, which is electrically actuated by a band actuator 138, and which includes a balloon 134 fluidly connected to a tube 142;
FIG. 10 is a side view of third gastric band embodiment 126;
FIG. 11 is a top view of a fourth gastric band embodiment 150 not according to the invention, which includes a first extendable element 160, a second extendable element 162, a first contractable element 164, and a second contractible element 166;
FIG. 12 is a side view of fourth gastric band embodiment 150;
FIG. 13 is an enlarged view of extendable element 160;
FIG. 14 is an enlarged view of contractable element 164;
FIG. 15 is a top view of a fifth gastric band embodiment 230 not according to the invention, which is electrically actuated by a band actuator 252, and which includes a cushion 232 forming an enclosure 240;
FIG.16 is a top view of cushion 232 in a straight configuration and containing a band 250;
FIG. 17 is a side view of cushion 232 in a straight configuration and showing a plurality of pillows 242;
FIG. 18 is a cross sectional view of cushion 232 and band 250;
FIG. 19 is a top view of a sixth gastric band embodiment 260 not according to the invention, which is electrically actuated by a band actuator 278, and which includes a plurality of bags 268 attached to a band 262;
FIG. 20 is a side view of band 262 in a straight configuration;
FIG. 21 is an enlarged, cut away view of first bag 272, revealing a multiplicity of beads 276;
FIG. 22 is a top view of an seventh gastric band embodiment 290 not according to the invention, which is electrically actuated by a band actuator 304, and which includes a tubular mesh 294 slidably attached to a band 292, shown in a loosened configuration;
FIG. 23 is a top view of seventh gastric band embodiment 290, shown in a tightened configuration;
FIG. 24 is a top view of a eighth gastric band embodiment 312 not according to the invention, which includes a cushioning material 324 containing a clamp element 322, shown in an open configuration;
FIG. 25 is a top view of eighth gastric band embodiment 312 shown in a clamping configuration;
FIG. 26 is a cross sectional view of band 314 of eighth gastric band embodiment 312;
FIG. 27 is a schematic diagram of a control system 400, which may be adapted for use with the previous gastric band embodiments shown in FIGS. 1-26;

### Detailed Description of the Invention

The drawings of the adjustable gastric band embodiments shown in the accompanying figures are not to scale.

The present invention is an adjustable gastric band system, which we also refer to as a food intake restriction device, and which includes an adjustable gastric band, or simply, a gastric band. The gastric band is mechanically adjustable or non-inflatable, meaning that a transcutaneous needle injection of a fluid is not required to change the restriction of the gastric band on the stomach or esophagus.

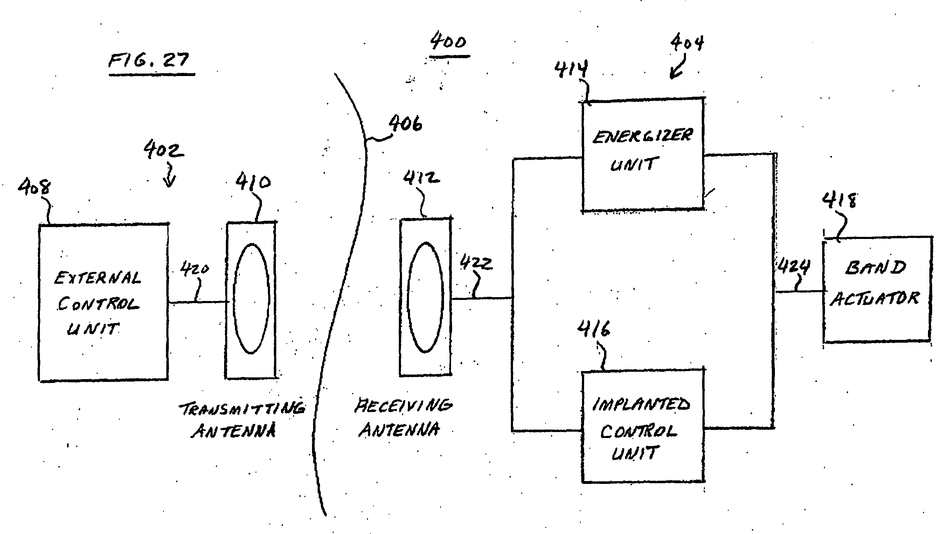

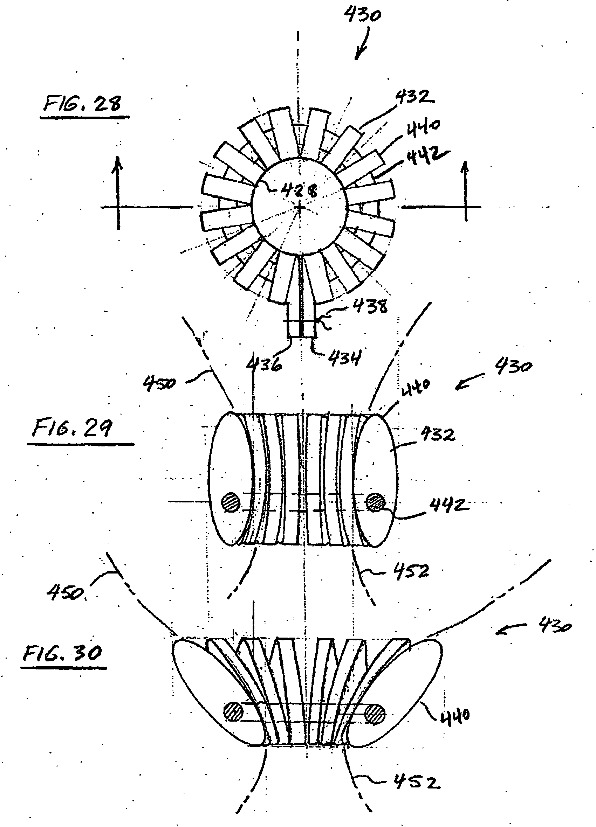

Several of the gastric band embodiments disclosed herein require electrical actuation via transcutaneous energy transmission means. Referring now to the drawings, FIG. 27 is a schematic diagram of a control system 400 that may be adapted for use with each of the electromechanically actuated, adjustable, gastric band embodiments. Control system 400 allows an operator to controllably transmit energy from outside of the patient to the inside of the patient. U.S. Patent number 6,067,991 ("Mechanical Food Intake Restriction Device," inventor Peter Forsell, issued May 30, 2000) provides a detailed description of this type of control system, and is incorporated herein for reference. In general, control system 400 includes an external portion 402 comprising an external control unit 408 electrically connected by an external cable 420 to a transmitting antenna 410. Control system further includes an implanted portion 404 comprising a receiving antenna 412 electrically connected by a first conductor 422 to an energizer unit 414 and an implanted control unit 416. A second conductor 424 electrically connects energizer unit 414 and implanted control unit 416 to a band actuator 418. Band actuator 418 may include any one of numerous means of transmitting power to an adjustable gastric band, as will be described.

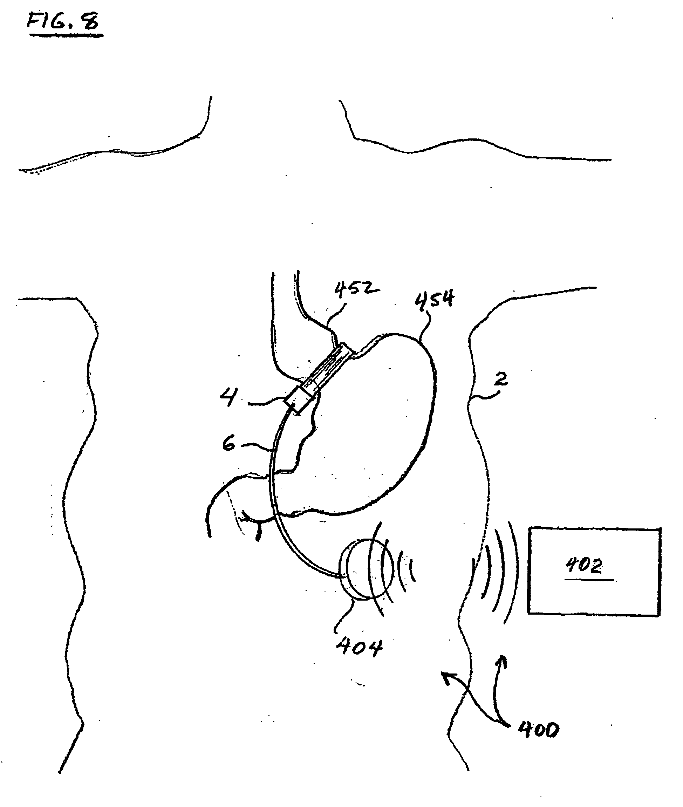

FIG. 8 is a representation of control system 400, showing implanted portion 404 in the patient 2 and connected by a cable 6 to an adjustable gastric band 4 positioned around the stomach 454. The operator uses external portion 402 to controllably transmit energy transcutaneously to implanted portion 404. The location of implanted portion 404 may be in various parts of the body, but generally within range of external portion 402. In FIG. 8, implanted portion 404 is subcutaneously implanted in the lower abdomen of the patient.

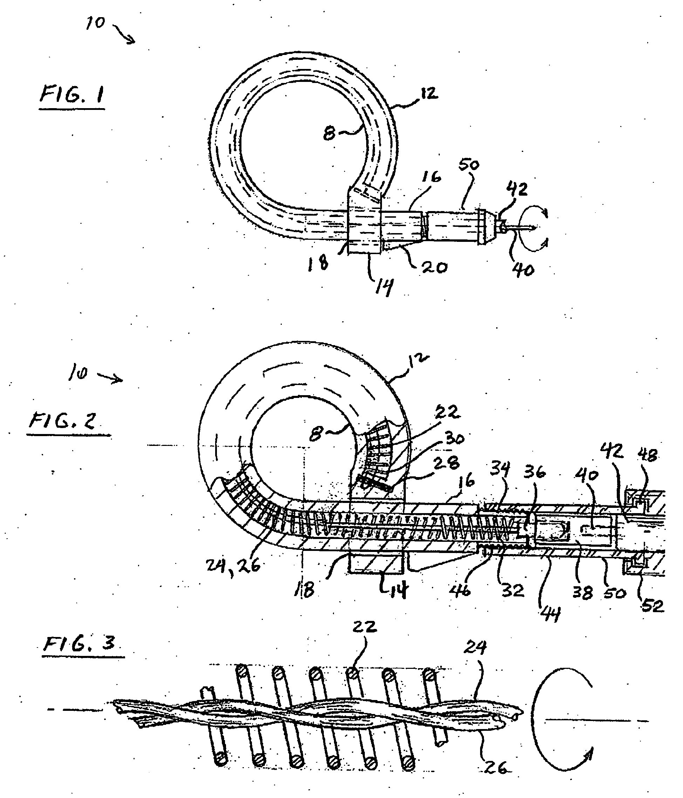

FIGS. 1, 2, and 3 show a first gastric band embodiment 10 that includes a band 12, which a surgeon may implant in a patient to form an enclosure 8 to restrict food intake through the stomach. FIG. 1 is a top view of first gastric band embodiment 10, showing band 12 with a second end 16 retained in an opening 18 of a first end 14 by a latch 20. Band 12 generally is a thick walled, flexible tube made of a soft, elastic, biocompatible material such as silicone or polyurethane rubber. The surgeon may laparoscopically introduce band 12 into the patient while band 12 is in a straight configuration (not shown.) A flexible shaft connector 50 releasably engages a flexible shaft 42 to band 12. Flexible shaft 42 includes a torsion cable 40. Flexible shaft 42 has a suitable length to extend to another location within the patient's body to implanted control unit 416 (FIG. 27.)

FIG 2 is also a top view of first gastric band embodiment 10. A portion of band 12 is cut away to reveal an extendable shield 22, which may be a compression spring, preferably made from a non-magnetic material such as a titanium alloy or a rigid plastic in order to be MRI compatible. Extendable shield 22 has a first end 30 located in first end 14 of band 12 and a second end 32 attached to a threaded bushing 34, which abuts against second end 16. Extendable shield 22 lengthens as enclosure 8 increases and contains a first strand 24 and a second strand 26. Strand termination 28 anchors first strand 24 and second strand 26 in first end 14 of band 12. First strand 24 and second strand 26 attach to a key 36 that operationally engages into a socket 38 within a coupler 44 of flexible shaft connector 50 for transmitting rotation from torque cable 40 to first strand 24 and second strand 26. A retainer 52 attached to flexible shaft 42 rotatably retains a second end 48 of coupler 44. While flexible shaft connector 50 is disengaged from band 12, a surgeon may introduce implant band 12 into a patient. Once the surgeon has placed band 12 around the stomach, the surgeon may connect flexible shaft connector 50 to band 12 by rotating coupler 44 onto threaded bushing 34.

FIG. 3 is an enlarged view of extendable shield 22, which contains first strand 24 and second strand 26. As first strand 24 and second strand 26 are twisted together during actuation of band 12, the length of band 12 shortens, thus reducing the size of enclosure 8. By controllably applying rotation in either direction to band 12, the surgeon may adjust the size of enclosure 8 in either direction.

Flexible shaft 42 extends to an electrical motor driven, band actuator (not shown) in another portion of the patient's body, and implanted subcutaneously to be within range of the energy transmission provided by control system 400 (FIG. 27.)

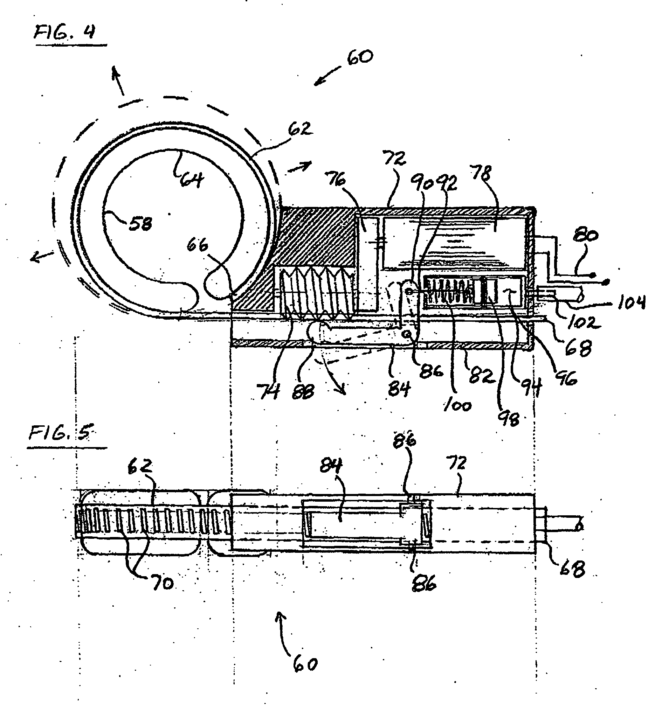

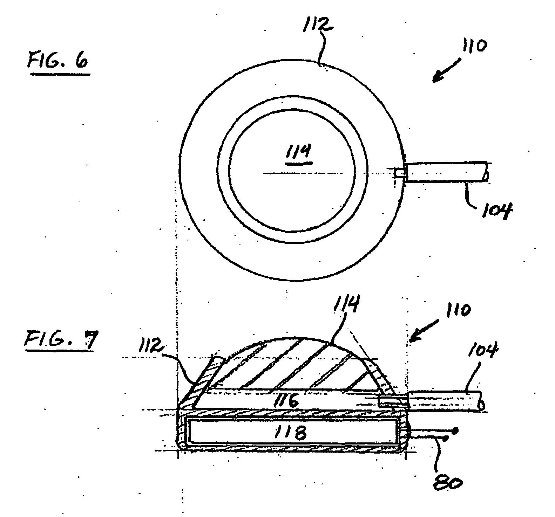

FIGS. 4 and 5 show a second gastric band embodiment 60 not according to the invention. A band 62 has a first end 66 and a second end 68 and forms an enclosure 58 to restrict food intake through the stomach of a patient. Band 62 is made of a spring-like, non-magnetic (for MRI compatibility) material and is in a straight configuration when in a unconstrained mode. A cushion 64 is made of a soft, biocompatible material such as silicone and attaches to the inside of band 62 to interface with the stomach tissue. First end 66 of band 62 attaches to a band actuator 72. Second end 68 of band 62 inserts into band actuator 72 after the surgeon has placed band 62 around the stomach. Band actuator 72 comprises a motor 78, which drives a pinion 74 through a transmission 76. Electrical conductors 80 electrically connect motor 78 to an implantable control port 110, which is shown in FIGS. 6 and 7. A control unit 118 within implantable control port 110 is analogous to implantable portion 404 of control system 400 shown in FIG. 27. Pinion 74 operationally engages a plurality of slots 70 of band 62. When motor 78 rotates in a first direction, enclosure 58 reduces in diameter; when motor 78 rotates in an opposite second direction, enclosure 58 increases in diameter. The surgeon may therefore control the amount of restriction to food intake through the stomach.

FIGS. 4 and 5 also show a release mechanism 94 inside of band actuator 72. Release mechanism 94 comprises a lever 84 having an axle 86, a first end 88, and a second end 90. When in an engaging mode, first end 88 bears against band 62, thus maintaining operational engagement of band 62 with pinion 74. When in a releasing mode, first end 88 is swung away about axle 86 from band 62, thus allowing band 62 to be disengaged from pinion 74. Release mechanism 94 further comprises a piston 98 that attaches via a link 92 to second end 90 of lever 84. A spring 100 normally urges piston 98 to move in a direction that causes lever 84 to be in the engaging mode. A chamber 96 containing piston 98 fluidly attaches to a tube 104 via a fitting 102. Tube 104 fluidly connects to a reservoir 116 formed within a housing 112 of implantable control port 110, shown in FIGS. 6 and 8. Implantable control port 110 further includes a septum 114 made of silicone and which is needle penetrable so that a surgeon may inject a fluid into reservoir 116 and actuate piston 98 of release mechanism 94. Implantable control port 110 may be implanted subcutaneously in a patient to be within the transcutaneous energy transmission range, and to allow the surgeon to access septum 114 to inject a fluid into reservoir 116. Release mechanism 94 allows non-surgical release of the constriction of band 62 on the stomach in the event of electromechanical failure.

Release mechanism 94 is not limited to hydraulic actuation as described for FIG. 4, but may incorporate a pneumatic, electrical, or other type of actuation. In addition, release mechanism 94 may be used with gastric bands that may be actuated with devices other than electric motors. For example, release mechanism 94 may be used with a gastric band that is inflatable with a fluid such as saline.

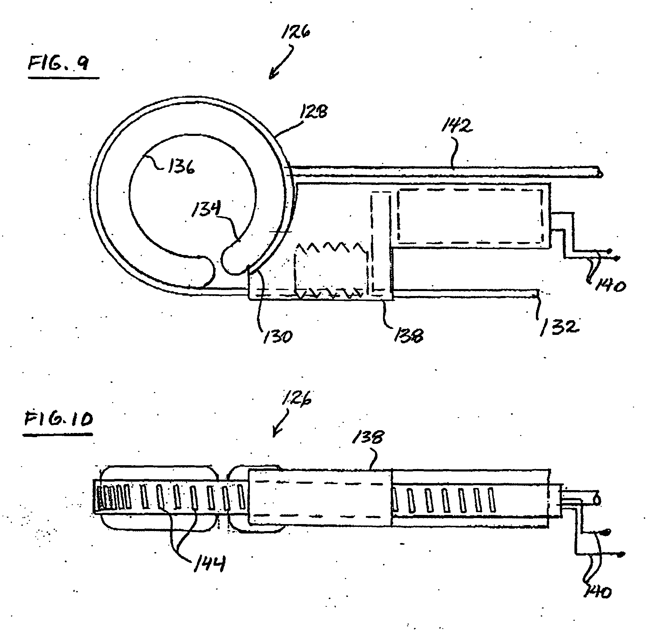

FIGS. 9 and 10 depict a third gastric band embodiment 126 not according to the invention. FIG. 9 is a top view of third gastric band embodiment 126 comprising a band 128 forming an enclosure 136 for restricting food intake through the stomach of a patient. A balloon 136 made of silicone or other elastic, biocompatible material, attaches to the inside of band 128 to form the tissue interface. A band actuator 138 provides a primary means of band actuation electromechanically. A tube 142 fluidly connected to balloon 134 provides a secondary means of band actuation, which a surgeon may use to replace or enhance the primary means. Band 128 includes a first end 130 attached to band actuator 138 and a second end 132 that inserts into band actuator 138 after the surgeon has placed band 128 around the stomach. As for second gastric band embodiment 60 shown in FIG. 4, band actuator 138 operationally engages with a plurality of slots 144 of band 128 to tighten or loosen band 128 around the stomach. Third gastric band embodiment 126 also fluidly and electrically (via conductors 140) connects to implantable control port 110 (FIGS 6 and 8) which may be planted subcutaneously in another location in the patient's body. The surgeon may inject a fluid such as saline into balloon 136 during initial surgical placement, or at anytime after implantation, to provide a cushion interface on the stomach, or to make slight adjustments to the amount of constriction on the stomach. To avoid needle injections during follow-up procedures, the surgeon may adjust the constriction using the primary, electromechanical means. The secondary, fluidic means provides system redundancy that may allow a patient to continue treatment for obesity without surgical intervention, should an electromechanical failure occur. The secondary, fluidic means also allows a surgeon to release some of the constriction on the stomach non-surgically in the event of electromechanical failure.

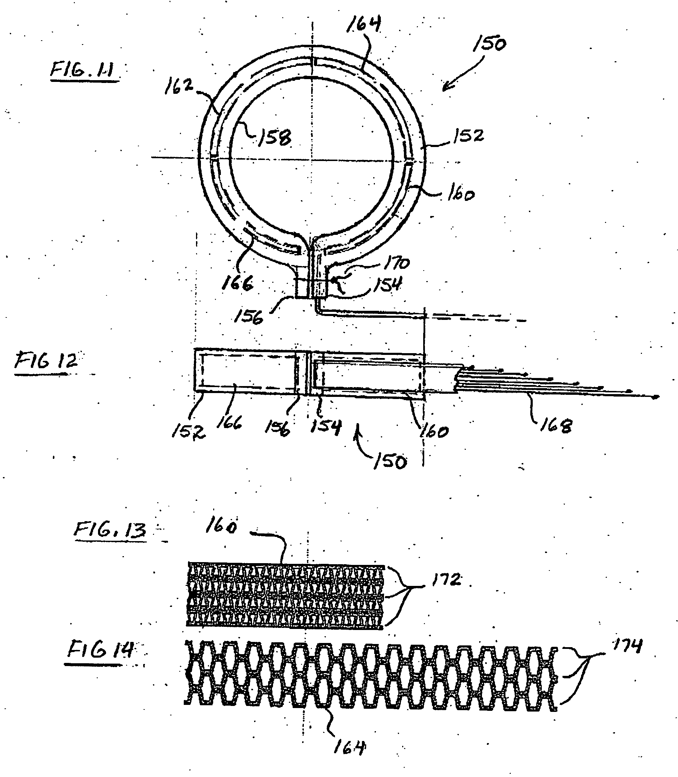

FIG. 11 is a top view and FIG. 12 is a side view of a fourth gastric band embodiment 150 not according to the invention, generally comprising a band 152 forming an enclosure 158 for restricting food intake through the stomach of a patient. Band 152 has a first end 154 and a second end 156 that a surgeon ties together with a suture 170 after placing band 152 around the stomach of the patient. Band 152 is made of a soft, elastic, biocompatible material such as silicone rubber. Embedded inside of band 152 is a first extendable element 160 (FIG. 13) and a second extendable element 162 alternately positioned in a circular pattern with a first contractable element 164 (FIG. 14) and a second contractable element 166. Elements 160, 162, 164, and 166 are physically connected in series (end-to-end) but are electrically isolated from the each other, and each electrically connects via conductors 168 to implanted portion 404 of control system 400 (FIG. 27.) The surgeon may controllable apply electrical energy selectively to one or more of elements 160, 162, 164, and 166 to loosen or tighten band 152 around the stomach. Band 152 may have many additional, shorter elements in order to generate finite band adjustments that are sufficiently small for the application.

Contractable elements 164 and 166, and extendable elements 160 and 162, are made of a shape memory metal such as Nitinol. Components made of Nitinol have special properties that are well know to those skilled in the art. Generally, a component may be fabricated from Nitinol at a low temperature into a first configuration, when the material is in a relatively soft and malleable, martinsitic state. Then the component may be heated to a very high temperature, such as 1000 degrees C, to an austenitic state, and shape set to a desired, second configuration. When the component cools, it transitions back to the martinisitic shape and may be easily shaped into the first configuration again or into a third configuration. When electrical current is passed through the component, the material heats. Sufficient current heats the component above an austenitic start temperature, at which point the component begins to transition to the austenitic state, and to deform to the second configuration. In addition to this shape memory effect, Nitinol also exhibits superelasticity properties.

As shown in FIGS. 13 and 14, extendable elements 160, 162 and contractible elements 164,166 comprise a plurality of spring-like beam structures arrayed into a one-piece mesh. Extendable element 160, which is identical to extendable element 162, is shape set and finished while in the high temperature, austenitic state to a long configuration such as shown in FIG. 13. Extendable element 160 is then shape set at the low temperature, soft, martinsitic state to the shortened configuration shown in FIG. 13. Contractible element 164, which is identical to contractible element 166, is shape set and finished while at the high temperature, austenitic state to the short configuration shown in FIG.13, and then shape set while in the low temperature, martinsitic state to the lengthened configuration shown in FIG.14. The austenitic start temperature is approximately 60 degrees C so that band 152 maintains an initial configuration in the patient's body until controllably actuated by the surgeon. Extendable elements 160, 162 include tabs 172 to function as mechanical and electrical connections. Contractible elements 164, 166 also include tabs 174 to function also as mechanical and electrical connections.

Once implanted and secured around the stomach, band 152 is initially loose around the stomach. The surgeon then may controllably actuate band 152 transcutaneously using control system 400 to send electrical current through contractable element 164, which heats above the austenitic temperature and shortens to a predetermined length. Control system 400 may be programmed to sequentially actuate additional contractable elements until enclosure 158 has reached a predetermined diameter, thus providing the desired constriction on the stomach. If band 152 contained many more than two contractile elements as in the present embodiment, then control system 400 preferably would sequentially energize non-adjacent elements to prevent localized heat build-up. Similarly, if the surgeon desired to loosen the band, the surgeon may use control system 400 to controllably actuate extendable elements 160 and 162, thus increasing the diameter of enclosure 158 in predetermined increments. While implanted and at body temperature, all of elements 160, 162, 164, and 164 would be in the soft, martinsitic state and would easily deform plastically in case the patient attempted to swallow something larger than medically advisable. The contractable elements could also be made to deform (lengthen) more easily than the extendable elements, so that in such cases, the contractable elements would stretch rather than the extendable elements. In a follow-up procedure, therefore, the surgeon could re-actuate the contractable elements back to the predetermined length to provide the desired constriction on the stomach.

In a variation of fourth gastric embodiment 180, extendable element 160 and contractible element 164 may be assembled side-by-side, or in parallel, as a single component that both extends and contracts upon actuation. When extendable element 160 is electrically actuated, the extension force as it changes to the stiff, austenitic state easily overcomes the force required to stretch contractible element 164, which is in the relatively soft, martinisitic state. Similarly, when contractible element 164 is actuated to contract, the contraction force easily overcomes the force required to contract extendable element 160. Extendable element 162 and contractible element 166 may also be assembled in parallel as a single component that both extends and contracts upon actuation. A thin insulation strip may be assembled between the parallel contractible and extendable elements within the single component to reduce heat transfer from the actuated element to the passive element. A plurality of small, extendable-contractible components may be joined in series like a chain and embedded into band 152, and electrically connected by a conductor array to implanted portion 402 of control system 400. By having a plurality of extendable-contractible components, control system 400 may be programmed to have finer, predetermined, incremental adjustments of the restriction on the stomach.

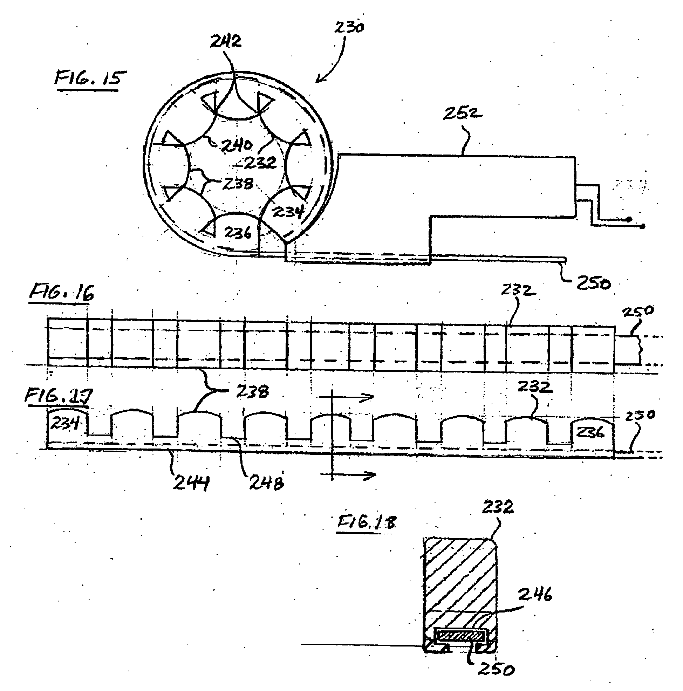

FIGS. 15, 16, 17, and 18 show a fifth gastric band embodiment 230 not according to the invention for restricting food intake through the stomach. FIG. 15 is a top view of fifth gastric band embodiment 230, comprising a band 250 forming an enclosure 240. Band 250 attaches to and is actuated by a band actuator 252 in the same fashion as for the third gastric band embodiment 126 shown in FIG. 9. A plurality of pillows 238 slidably attaches to the inside of band 250. Pillows 238 are made of a soft, compliant, biocompatible material such as silicone rubber. FIG. 16 is a top view and FIG. 17 is a side view of plurality of pillows 238, which includes a first end 234 and a second end 236. Pillows 238 are equally spaced apart by a span 248 and attached to or integrally molded onto a base 244. The shape of each of pillows 238 may vary, but in general, pillows 238 form a continuous perimeter (without gaps) around enclosure 240 when the surgeon positions band 250 around the stomach. As the surgeon adjusts band 250 to tighten around the stomach, pillows 238 deform and bulge primarily in a radial, inward direction. FIG. 18 is a cross sectional view of band 250 slidably retained in a channel 246 of pillows 238.

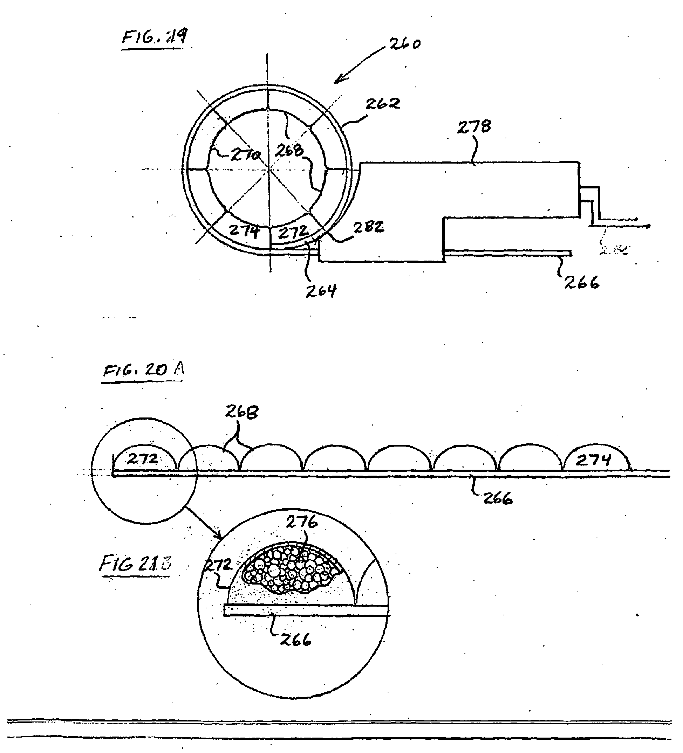

FIGS. 19 is a top view of a sixth gastric band embodiment 260, not according to the invention comprising a band actuator 278 and a band 266 forming an enclosure 270 for restricting food intake through the stomach. Band 266 and band actuator 278 are similar in design and operation to band 250 and band actuator 252 of the fifth gastric band embodiment shown in FIG.15. A plurality of bags 268 attaches to the inside of band 266 to form the tissue interface. FIG. 20 shows a portion of band 266 in a straight configuration and including a first bag 272 and a second bag 274. Each of bags 268 is made of a thin-wall, tough plastic material such as polyethylene, or of a thick-wall, soft, elastomeric material such as silicone rubber. FIG 21 is an enlarged view of first bag 272, which contains a multiplicity of beads 276. Beads 276 are generally spherically shaped and have a smooth surface finish so that bags 268 may conform readily to the stomach tissue. The exact shape and surface finish of beads 276 may be selected to provide the desired deformability characteristics of bags 268 for interfacing bodily tissue. Beads 276 may vary in size, and have a diameter not limited to, but approximately in the range, of 0.5 to 2.0 mm. Beads 276 may be made of a hard, biocompatible metal, ceramic, or plastic.

Alternatively, bags 268 may be made of a water permeable material and filled with a hydrogel composition such as 98-99% hydrolyzed, polyvinyl alcohol (PVA). Initially bags 268 would be flat and relatively flaccid to facilitate introduction into the body and placement around the stomach. Because of the osmotic pressure difference between body tissue fluid surrounding bags 268 and the hydrogel composition, fluids would pass into bags 268 and become absorbed by the hydrogel composition until equilibrium was reached. Consequently, the hydrogel composition would swell to many times the original, dehydrated volume, thus causing bags 268 to fill and provide soft and compliant tissue interfaces. The size of each bag at osmotic equilibrium may be predetermined depending on the exact mass and composition of the hydrogel material inside of each of bags 268.

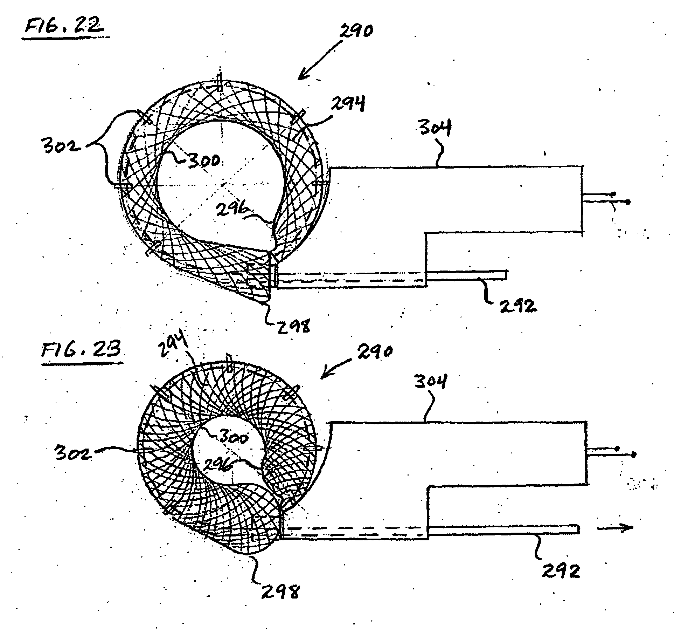

FIG. 22 and FIG. 23 show a seventh gastric band embodiment 290 not according to the invention, comprising a band actuator 304 and a band 292 forming an enclosure 300 for restricting food intake through the stomach. Band 292 and band actuator 304 are similar in design and operation to band 250 and band actuator 252 of the sixth gastric band embodiment shown in FIG. 15. A tubular mesh 294 covers band 250 and has a first end 296 and a second end 298 attached to band 250. A plurality of sliding attachments 302 retains tubular mesh 294 against the outside of band 250. Tubular mesh 294 comprises a plurality of biocompatible, braided monofilaments made of an extruded plastic such as polyester or polyethylene. Similar products are available in the electrical industry for bundling wire cables and the like. Tubular mesh 294 is constructed so that when compression force is applied to the opposite ends in the longitudinal direction, and tubular mesh 294 is constrained from buckling, tubular mesh 294 shortens in length and grows in cross-sectional area. FIG. 22 is a top view of seventh gastric band embodiment 290 in a loose configuration; FIG. 23 is a top view in a tightened configuration, showing how actuating band 292 causes enclosure 300 to decrease in area as tubular mesh 294 bulges radially inward. Tubular mesh 294 optionally may be covered with a soft, compliant, elastic material such silicone to reduce trauma to the stomach tissue. If covered with such a material, and if first end 296 and second end 298 are sealed, tubular mesh 294 may also be filled with a fluid, beads or a hydrogel composition, as described for the embodiment shown in FIG. 21, in order to obtain desired tissue interface characteristics.

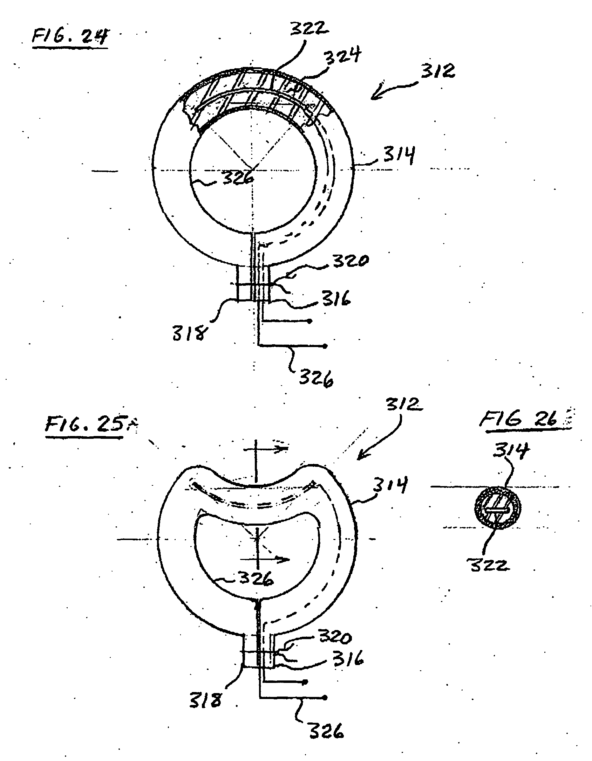

FIG. 24 is a top view of a eighth gastric band embodiment 312, not according to the invention, comprising a band 314 forming an enclosure 326 for restricting food intake through the stomach. Band 314 includes a filler 324, which may be made of a soft, biocompatible, elastic material such as silicone. Band 314 includes a first end 316 and a second end 318 held together with a suture 320 after placement around the stomach, so that enclosure 326 is approximately circular and loosely surrounding the stomach. In FIG. 24, clamp element 322 is in a first configuration having a radius of curvature approximately equal to the centerline radius of band 314. A surgeon may use control system 400 (FIG. 27) to electrically actuate clamp element 322 to change to a second configuration as shown in FIG. 25, in which enclosure 326 assumes approximately a D-shape having a reduced area. Conductors 326 electrically connect 322 to implanted portion 404 of control system 400 (FIG 27.) FIG. 26 is a cross-sectional view of band 314 containing clamping element 322. Clamping element 322 may be actuated again to resume the first configuration as shown in FIG. 25.

Although only one clamping element 322 is shown in FIG. 25, it is possible to have more than one clamping element, each independently actuatable via control system 400 so that the size of enclosure 326 may be reduced or increased in small, predetermined increments.

Clamping element 322 comprises a pair of shape memory metal strips separated by a thin, flexible strip of insulating material such as PTFE, Teflon, or the like. The shape memory metal may be Nitinol that changes from a soft, pliable martinsitic structure to a hard, stiff austenitic structure at a temperature of approximately 60 degrees C. Two Nitinol strips are shape set into a curved configuration as shown in FIG. 24 while at a very high temperature. When the curved strips cool back to the martinsitic structure, the strip still maintains the shape set curvature, although each is again soft and pliable. Next, one of the Nitinol strips is bent to the mirror-reflection curvature and then assembled to the other Nitinol strip with the insulation strip in between. A pair of electrical conductors is electrically connected to each strip so that they may be independently energized with electric current. When one of the strips is energized, that strip heats to a temperature slightly higher than the austenitic start temperature (60 degrees C, for example) and curves to the austenitic shape set configuration, if it was not already in that configuration. Due to the nature of Nitinol, the force of one electrically actuated strip to bend to the austenitic shape set configuration is greater than the bending resistance of the non-actuated, soft, martinisitic strip. Therefore, when one strip is actuated and deforms into a new configuration, the non-actuated strip passively bends to the same configuration as the actuated strip.

It will be apparent, therefore, to one skilled in the art, that numerous clamp elements, each comprised of a pair of shape set Nitinol strips, may be constructed into band 314 and independently actuated by control system 400 (FIG. 27) to move radially inward or outward from the center of enclosure 326. The numerous clamp elements may be positioned end-to-end along the length of band 314. A surgeon may program control system 400 to only actuate a certain number of these clamp elements, in order to achieve a desired enclosure size and stomach restriction. If necessary, the enclosure may be further increased in size or decreased, depending on the needs of the patient, in a controllable fashion.

It will become readily apparent to those skilled in the art that the above invention has equally applicability to other types of implantable bands. For example, bands are used for the treatment of fecal incontinence. One such band is described in U.S. Patent 6,461,292 Bands can also be used to treat urinary incontinence. One such band is described in U.S. Patent Application 2003/0105385. Bands can also be used to treat heartburn and/or acid reflux. One such band is described in U.S. Patent 6,470,892. . Bands can also be used to treat impotence. One such band is described in U.S. Patent Application 2003/0114729.

The embodiments disclosed herein are described but not limited to the application of restricting food intake through the stomach. The present invention, however, may also be applicable to other therapeutic purposes on the stomach, esophagus, or other organs of the body of a patient.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. A food intake restriction device (10) for surgical application in the abdomen of a patient for forming a stoma opening in the stomach or esophagus of the patient, said device comprising:
- an elongated restriction member (12);
- forming means for forming said restriction member (12) into at least a substantially closed loop around the stomach or the esophagus, said loop defining an enclosure (8);
- a control system (416) for post-operative, non-invasive, mechanical adjustment of said restriction member (12) to change the size of said enclosure (8); **characterized in that**:
said restriction member (12) contains a pair of substantially coextensive strands (24, 26), said pair of strands having a first, non-rotatable end and a second, rotatable end whereby an operator may rotate said strands (24, 26) to twist them together in a first direction to decrease the size of said enclosure (8) and in a second direction to increase the size of said enclosure (8).

## Patentansprüche

1. Einrichtung (10) zur Begrenzung der Nahrungsaufnahme zur chirurgischen Anwendung im Bauchbereich eines Patienten, um eine Stoma-Öffnung im Magen oder in der Speiseröhre zu bilden; umfassend:
- ein längliches Begrenzungsglied (12),
- eine Formeinrichtung zum Formen des Begrenzungsglieds (12) in mindestens eine im Wesentlichen geschlossene Schlinge um den Magen oder um die Speiseröhre, wobei die Schlinge eine Einschließung (8) definiert;
- ein Steuerungs- und/oder Regelungssystem (4, 6) zum postoperativen, nicht invasiven, mechanischen Einstellen des Begrenzungsglieds (12), um die Größe der Einschließung (8) zu verändern, **dadurch gekennzeichnet, dass**
das Begrenzungsglied (12) ein Paar im Wesentlichen gleich lange Stränge (24, 26) enthält, die ein erstes, nicht drehbares Ende und ein zweites, drehbares Ende haben, wobei eine Bedienperson die Stränge (24, 26) drehen kann, um sie in einer ersten Richtung zu verdrehen, so dass die Einschließung (8) verkleinert wird und um sie in einer zweiten Richtung zu verdrehen, so dass der eingeschlossene Bereich (8) vergrößert wird.

## Revendications

1. Dispositif de limitation d'entrée de nourriture (10) pour une application chirurgicale dans l'abdomen d'un patient pour former une ouverture de stomie dans l'estomac ou l'oesophage du patient, ledit dispositif comprenant :
➢ un élément de limitation allongé (12) ;
➢ des moyens de formation pour former ledit élément de limitation (12) en au moins une boucle sensiblement fermée autour de l'estomac ou de l'oesophage, ladite boucle définissant une enceinte (8) ;
➢ un système de commande (416) pour l'ajustement mécanique, non invasif, post-opératoire dudit élément de limitation (12) afin de changer la taille de ladite enceinte (8), **caractérisé en ce qui :**
➢ ledit élément de limitation (12) contient une paire de brins (24, 26) sensiblement coextensifs, ladite paire de brins ayant une première extrémité non rotative et une seconde extrémité rotative, moyennant quoi un opérateur peut faire tourner lesdits brins (24, 26) pour les torsader dans une première direction afin de diminuer la taille de ladite enceinte (8) et dans une seconde direction afin d'augmenter la taille de ladite enceinte (8).
